# FASCICULE DE BREVET EUROPEEN

(11) **EP 1 403 264 B1**
(45) Date de publication et mention de la délivrance du brevet: **29.12.2004**
(21) Numéro de dépôt: 03292317.9
(22) Date de dépôt: 22.09.2003
(51) Int. Cl.: C07D 333/38

(54) **Procédé de synthèse industriel du diester méthylique de l'acide 5-amino-3-carboxyméthyl-4-cyano-2-thiophénecarboxylique**
Verfahren zur industriellen Herstellung von 5-Amino-3-carboxymethyl-4-cyano-2-thiphencarboxydimethylester
Process for the industrial synthesis of 5-amino-3-carboxymethyl-4-cyano-2-thiophenecarboxydimethylester

(30) Priorité: 24.09.2002 FR 0211764
(43) Date de publication de la demande: 31.03.2004
(73) Titulaire: Les Laboratoires Servier, 92415 Courbevoie Cedex (FR)
(72) Inventeur: Vaysse-Ludot, Lucile, 76490 Saint Wandrille Rancon (FR); Lecouve, Jean-Pierre, 76600 Le Havre (FR); Langlois, Pascal, 76210 Saint Jean de La Neuville (FR)

(56) Documents cités:
- EP-A- 0 415 850
- WIERZBICKI, MICHEL ET AL: "Reactivity of 2-aminothiophenes. Application to synthesis of thieno[2,3-b]pyrroles" BULLETIN DE LA SOCIETE CHIMIQUE DE FRANCE (1975), (7-8, PT. 2), 1786-92, XP009008611

## Description

La présente invention concerne un procédé de synthèse industriel du diester méthylique de l'acide 5-amino-3-carboxyméthyl-4-cyano-2-thiophènecarboxylique, et son application à la production industrielle des sels bivalents de l'acide ranélique et de leurs hydrates.

Plus spécifiquement, la présente invention concerne un nouveau procédé de synthèse industriel du dérivé de formule (I) :

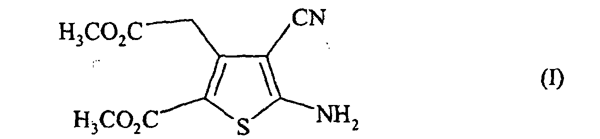

Le composé de formule (I) obtenu selon le procédé de l'invention est utile dans la synthèse de l'acide ranélique, de ses sels de strontium, de calcium ou de magnésium de formule (II) : 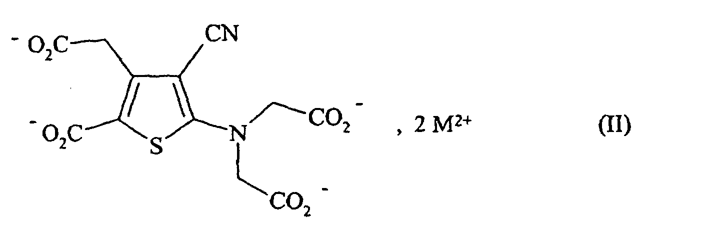 dans laquelle M représente le strontium, le calcium ou le magnésium,
et des hydrates desdits sels.

Les sels bivalents de l'acide ranélique possèdent des propriétés pharmacologiques et thérapeutiques très intéressantes, notamment des propriétés anti-ostéoporotiques remarquables, qui rendent ces composés utiles dans le traitement des maladies osseuses.

Les sels bivalents de l'acide ranélique, et plus particulièrement le ranélate de strontium, leur préparation et leur utilisation en thérapeutique ont été décrits dans le brevet européen EP 0415 850.

Ce brevet décrit l'accès au ranélate de strontium à partir du tétraester éthylique de formule (III) : 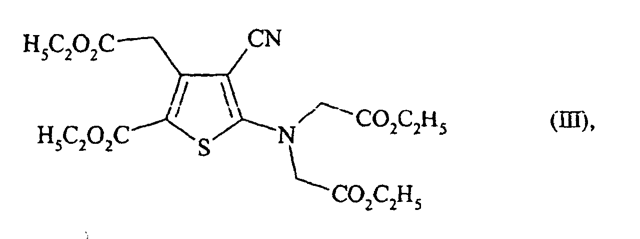 lui même accessible à partir du diester éthylique de formule (IV) :

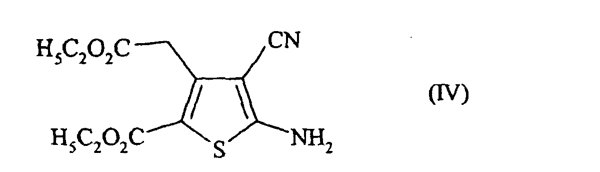

L'accès à l'intermédiaire de formule (IV) a été décrit dans la publication Bull. Soc. Chim. France 1975, pp. 1786-1792 et dans la publication J. Chem. Tech. Biotechnol. 1990, 47, pp. 39-46, par réaction entre le 3-oxoglutarate de diéthyle, le malononitrile et le soufre dans l'éthanol, en présence de morpholine ou de diéthylamine.

Ce procédé présente l'avantage d'utiliser des matières premières aisément accessibles, et d'être simple à mettre en oeuvre, mais, transposé à l'échelle de quelques centaines de kg, il ne permet pas d'obtenir le composé de formule (IV) avec un rendement supérieur à 70 %.

Or, compte-tenu de l'intérêt pharmaceutique du ranélate de strontium et des tonnages réalisés, il était important de pouvoir accéder à un diester de l'acide 5-amino-3-carboxyméthyl-4-cyano-2-thiophènecarboxylique de pureté excellente, avec un rendement au moins égal à 77 %, reproductible à l'échelle industrielle.

La Demanderesse a présentement mis au point un procédé de synthèse industriel performant, permettant d'obtenir le diester méthylique de formule (I) avec un rendement au moins égal à 77 % et une pureté supérieure à 97 %.

Le composé de formule (I) ainsi obtenu est particulièrement utile dans la synthèse de l'acide ranélique, de ses sels de strontium, de calcium ou de magnésium et des hydrates desdits sels, et plus particulièrement du ranélate de strontium et de ses hydrates. Il est alors mis en réaction avec un ester de l'acide bromoacétique, pour conduire au tétraester correspondant, qui est alors transformé en acide ranélique ou en l'un de ses sels de strontium, de calcium ou de magnésium.

Plus spécifiquement, la présente invention concerne un procédé de synthèse industriel du composé de formule (I),
caractérisé en ce que l'on met en réaction le 3-oxoglutarate de diméthyle de formule (V) : 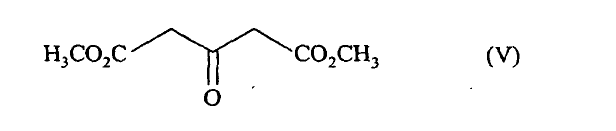 avec le malononitrile de formule (VI) : 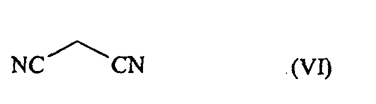 dans le méthanol,
en présence de morpholine en quantité supérieure à 0,95 mole par mole de composé de formule (V),
pour conduire au composé de formule (VII) : 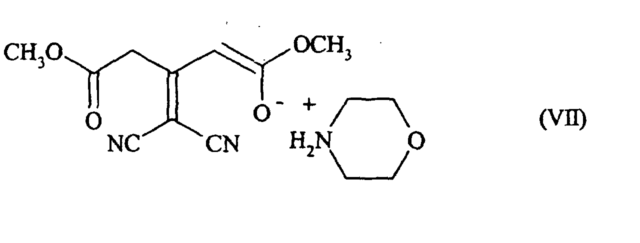 que l'on met ensuite en réaction avec du soufre en quantité supérieure à 0,95 mole par mole de composé de formule (V),
que l'on chauffe ensuite le mélange réactionnel au reflux,
et que l'on isole le composé de formule (I) ainsi obtenu par précipitation en présence d'eau, suivie d'une filtration.

Le procédé, ainsi amélioré par l'utilisation de ces conditions très spécifiques, et notamment par la formation intermédiaire du composé de formule (VII), éventuellement isolable, permet d'obtenir le composé de formule (I) avec une excellente pureté et un rendement reproductible à l'échelle de quelques centaines de kg et au moins égal à 77 %, ce qui représente un gain de rendement capital, compte-tenu des tonnages importants de ranélate de strontium produits.

De façon préférentielle, la quantité de méthanol est comprise entre 1 et 3 ml par gramme de composé de formule (V).

La température de réaction entre les composés de formule (V) et (VI) est de préférence inférieure à 50°C.

Le temps de réaction au reflux après addition du soufre est préférentiellement compris entre 1 h 30 et 3 h.

Le 5-amino-4-cyano-3-(2-méthoxy-2-oxoéthyl)-2-thiophènecarboxylate de méthyle de formule (I) et l'intermédiaire de formule (VII) sont des produits nouveaux, utiles comme intermédiaires de synthèse dans l'industrie chimique ou pharmaceutique, notamment dans la synthèse du ranélate de strontium, et font à ce titre partie intégrante de la présente invention.

Les exemples ci-dessous illustrent l'invention, mais ne la limitent en aucune façon.

### EXEMPLE 1 : 5-Amino-4-cyano-3-(2-méthoxy-2-oxoéthyl)-2-thiophènecarboxylate de méthyle.

Charger dans un réacteur 400 kg de 3-oxoglutarate de diméthyle, 158 kg de malononitrile et 560 1 de méthanol, puis, en maintenant la température du milieu réactionnel inférieure à 40°C, 199,6 kg de morpholine.

Charger ensuite 73,6 kg de soufre, puis amener le mélange au reflux.

Après 2 h de réaction, couper le reflux, ajouter de l'eau jusqu'à précipitation. Filtrer le précipité obtenu, le laver et le sécher.

Le 5-amino-4-cyano-3-(2-méthoxy-2-oxoéthyl)-2-thiophènecarboxylate de méthyle est ainsi obtenu avec un rendement de 77 % et une pureté chimique de 98 %.

### EXEMPLE 2 : 5-Amino-4-cyano-3-(2-méthoxy-2-oxoéthyl)-2-thiophènecarboxylate de méthyle.

Charger dans un réacteur 400 kg de 3-oxoglutarate de diméthyle, 158 kg de malononitrile et 560 1 de méthanol, puis, en maintenant la température du milieu réactionnel inférieure à 40°C, 199,6 kg de morpholine.

Le composé de formule (VII) ainsi obtenu, ou sel d'addition du 3-(dicyanométhylène)-5-hydroxy-5-méthoxy-4-penténoate de méthyle avec la morpholine, est isolé par filtration après refroidissement du milieu, puis mis en réaction dans le méthanol avec 73,6 kg de soufre.

Le mélange est ensuite amené au reflux.

Après 2 h de réaction, couper le reflux, ajouter de l'eau jusqu'à précipitation. Filtrer le précipité obtenu, le laver et le sécher.

## Revendications

1. Procédé de synthèse industriel du composé de formule (I) : 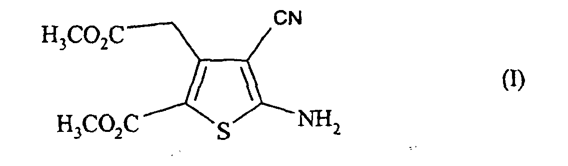 **caractérisé en ce que** l'on met en réaction le 3-oxoglutarate de diméthyle de formule (V) : 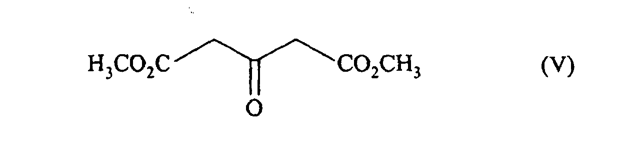 avec le malononitrile de formule (VI) : 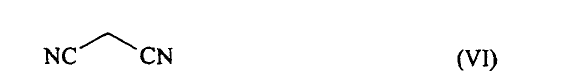 dans le méthanol,
en présence de morpholine en quantité supérieure à 0,95 mole par mole de composé de formule (V),
pour conduire au composé de formule (VII) : 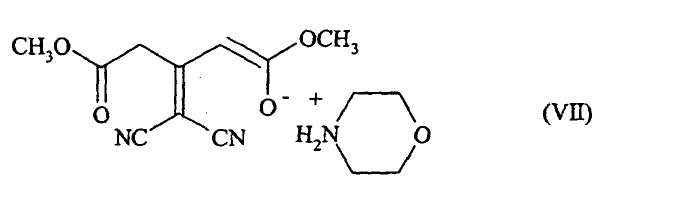 que l'on met ensuite en réaction avec du soufre en quantité supérieure à 0,95 mole par mole de composé de formule (V),
que l'on chauffe ensuite le mélange réactionnel au reflux,
et que l'on isole le composé de formule (I) ainsi obtenu par précipitation en présence d'eau, suivie d'une filtration.

2. Procédé de synthèse selon la revendication 1, **caractérisé en ce que** la quantité de méthanol est comprise entre 1 et 3 ml par gramme de composé de formule (V).

3. Procédé de synthèse selon l'une quelconque des revendications 1 ou 2, **caractérisé en ce que** la température de réaction entre les composés de formules (V) et (VI) est inférieure à 50°C.

4. Procédé de synthèse selon l'une quelconque des revendications 1 à 3, **caractérisé en ce que** le temps de réaction au reflux après addition du soufre est compris entre 1 h 30 et 3 h.

5. 5-Amino-4-cyano-3-(2-méthoxy-2-oxoéthyl)-2-thiophènecarboxylate de méthyle.

6. Composé de formule (VII) :

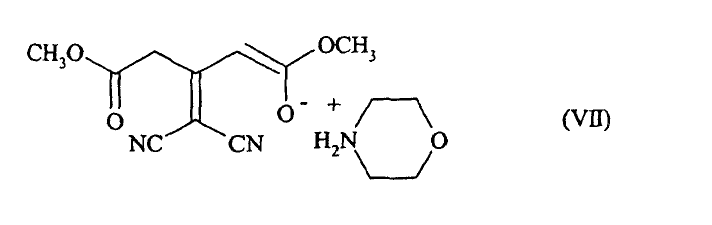

7. Procédé de synthèse de l'acide ranélique, de ses sels de strontium, de calcium ou de magnésium et des hydrates desdits sels, à partir d'un composé de formule (I) : 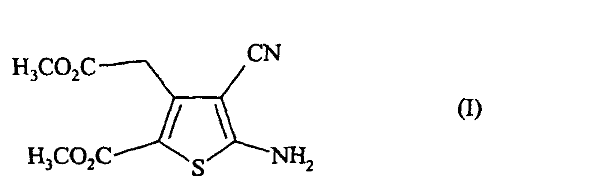 que l'on met en réaction avec un ester de l'acide bromoacétique, pour conduire au tétraester correspondant, que l'on transforme en acide ranélique ou en l'un de ses sels de strontium, de calcium ou de magnésium,
**caractérisé en ce que** le composé de formule (I) est obtenu par le procédé de synthèse selon l'une quelconque des revendications 1 à 4.

8. Procédé de synthèse du ranélate de strontium et de ses hydrates, à partir d'un composé de formule (I) : 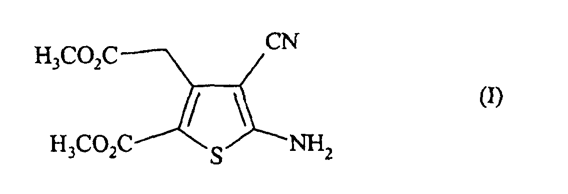 que l'on met en réaction avec un ester de l'acide bromoacétique, pour conduire au tétraester correspondant, que l'on transforme en ranélate de strontium,
**caractérisé en ce que** le composé de formule (I) est obtenu par le procédé de synthèse selon l'une quelconque des revendications 1 à 4.

## Claims

1. Process for the industrial synthesis of the compound of formula (1) : 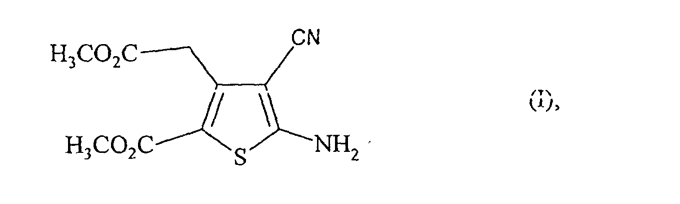 **characterised in that** dimethyl 3-oxoglutarate of formula (V) : 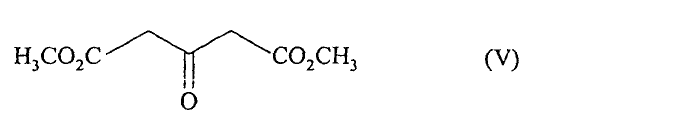 is reacted with malononitrile of formula (VI) : 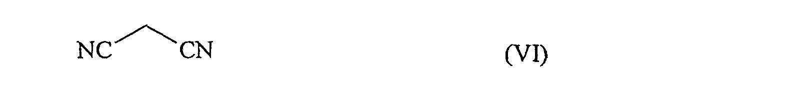 in methanol,
in the presence of morpholine in an amount greater than 0.95 mol per mol of compound of formula (V),
to yield the compound of formula (VII) : 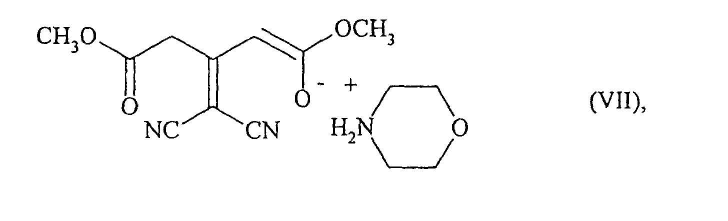 which is then reacted with sulphur in an amount greater than 0.95 mol per mol of compound of formula (V);
the reaction mixture is then heated at reflux;
and the compound of formula (I) thereby obtained is isolated by precipitation in the presence of water, followed by filtration.

2. Synthesis process according to claim 1, **characterised in that** the amount of methanol is from 1 to 3 ml per gram of compound of formuia (V).

3. Synthesis process according to either claim 1 or claim 2, **characterised in that** the temperature of reaction between the compounds of formulae (V) and (VI) is less than 50°C.

4. Synthesis process according to any one of claims 1 to 3, **characterised in that** the reaction time at reflux after addition of the sulphur is from 1 hour 30 minutes to 3 hours.

5. Methyl 5-amino-4-cyano-3-(2-methoxy-2-oxoethyl)-2-thiophenecarboxylate.

6. The compound of formula (VII) :

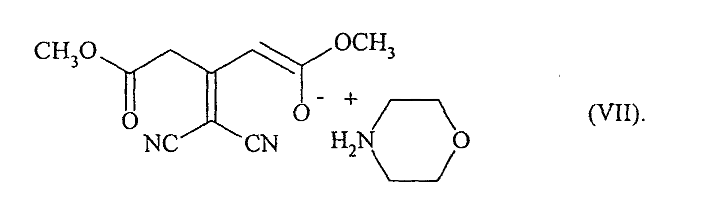

7. Process for the synthesis of ranelic acid, its strontium, calcium or magnesium salts and hydrates of the said salts, starting from a compound of formula (1) : 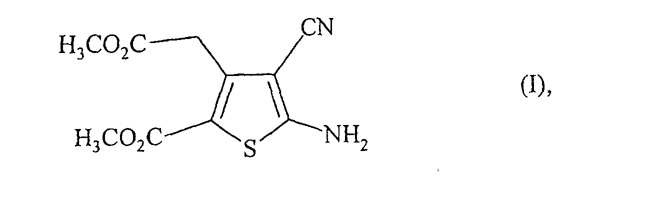 which is reacted with an ester of bromoacetic acid to yield the corresponding tetraester, which is converted into ranelic acid or a strontium, calcium or magnesium salt thereof,
**characterised in that** the compound of formula (I) is obtained by a synthesis process according to any one of claims 1 to 4.

8. Process for the synthesis of strontium ranelate and its hydrates, starting from a compound of formula (I) : 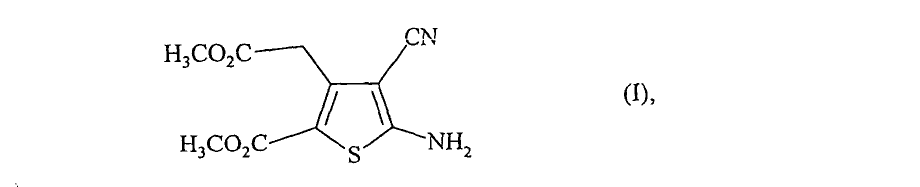 which is reacted with an ester of bromoacetic acid to yield the corresponding tetraester, which is converted into strontium ranelate,
**characterised in that** the compound of formula (I) is obtained by a synthesis process according to any one of claims 1 to 4.

## Patentansprüche

1. Verfahren zur industriellen Herstellung der Verbindung der Formel (I): 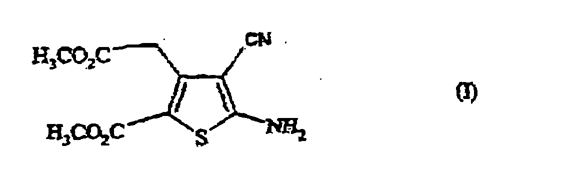 **dadurch gekennzeichnet, daß** man 3-Oxoglutarsäure-dimethylester der Formel (V): 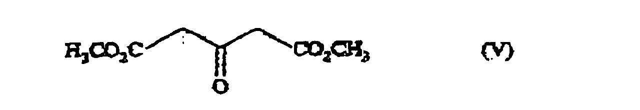 mit Malononitril der Formel (VI): 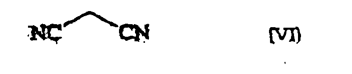 in Methanol
in Gegenwart von Morpholin in einer Menge von mehr als 0.95 Mol pro Mol der Verbindung der Formel (V) umsetzt.
zur Bildung der Verbindung der Formel (VII): 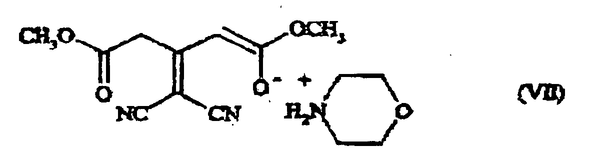 welche man anschließend mit Schwefel in einer Menge von mehr als 0,95 Mol pro Mol der Verbindung der Formel [V] umsetzt.
man anschließend die Reaktionsmischung zum Sieden am Rückfluß erhitzt und die in dieser Weise erhaltene Verbindung der Formel (I) durch Ausfällen in Gegenwart von Wasser. gefolgt von einer Filtration. isoliert.

2. Herstellungsverfahren nach Anspruch 1, **dadurch gekennzeichnet, daß** die Menge des Methanols zwischen 1 und 3 ml pro Gramm der Verbindung der Formel (V) liegt.

3. Herstellungsverfahren nach einem der Ansprüche 1 oder 2, **dadurch gekennzeichnet, daß** die Temperatur der Reaktion zwischen den Verbindungen der Formeln (V) und (VI) unterhalb 50°C liegt.

4. Herstellungsverfahren nach einem der Ansprüche 1 bis 3, **dadurch gekennzeichnet, daß** die Reaktionszeit am Rückfluß nach der Zugabe von Schwefel zwischen 1 Stunde 30 Minuten und 3 Stunden beträgt.

5. 5-Amino-4-cyano-3-(2-methoxy-2-oxoethyl)-2-thiophencarbonsäuremethylester.

6. Verbindung der Formel (VII):

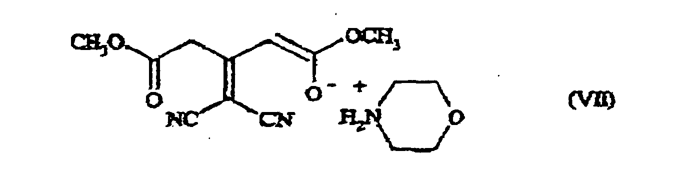

7. Verfahren zur Herstellung von Ranelinsäure, ihrer Strontium-, Calcium- oder Magnesiumsalze und den Hydraten dieser Salze, ausgehend von einer Verbindung der Formel (I): 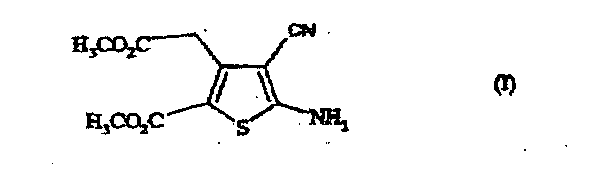 welche man mit einem Ester der Bromessigsäure umsetzt zur Bildung des entsprechenden Tetraesters, welche man in Ranelinsäure oder eines ihrer Strontium-. Calcium- oder Magnesiumsalze umwandelt.
**dadurch gekennzeichnet, daß** man die Verbindung der Formel (I) mit Hilfe des Herstellungsverfahrens gemäß einem der Ansprüche 1 bis 4 herstellt.

8. Verfahren zur Herstellung von Strontiumranelat und seinen Hydraten, ausgehend von einer Verbindung der Formel (I): 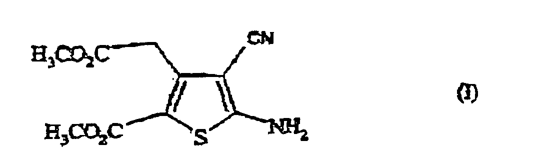 welche man mit einem Ester der Bromessigsäure umsetzt zur Bildung des entsprechenden Tetraesters, welchen man in Strontiumranelat umwandelt,
**dadurch gekennzeichnet, daß** man die Verbindung der Formel (I) mit Hilfe des Herstellungsverfahrens nach einem der Ansprüche 1 bis 4 herstellt.
